# EUROPEAN PATENT SPECIFICATION

(11) **EP 2 709 684 B1**
(45) Date of publication and mention of the grant of the patent: **25.05.2016**
(21) Application number: 11757689.2
(22) Date of filing: 01.06.2011
(51) Int. Cl.: A61L 27/22, A61L 27/34, A61L 31/10

(54) **SUBSTANCE ENHANCING BIOCOMPATIBILITY OF IMPLANTS WITH RECIPIENT BODY AND METHOD OF ITS PREPARATION.**
STOFF FÜR VERBESSERTE BIOKOMPATIBILITÄT VON IMPLANTATEN MIT EINEM AUFNAHMEKÖRPER UND VERFAHREN ZU SEINER HERSTELLUNG
SUBSTANCE POUR AMÉLIORER LA BIOCOMPATIBILITÉ DES IMPLANTS AVEC LE CORPS D'UN RECEVEUR ET SON PROCÉDÉ DE PRÉPARATION

(30) Priority: 18.05.2011 UA 2011006240
(43) Date of publication of application: 26.03.2014
(73) Proprietor: Co Ltd. "Mabela", Kiev 03035 (UA)
(72) Inventor: LAZARENKO, Oleg Mykolayevich, Kiev 03035 (UA); ALEKSYEYEVA, Tetiana Anatoliyivna, Kiev 01030 (UA)
(74) Representative: PATENTSERVIS Praha, a.s.
(86) International application number: PCT/UA2011/000045
(87) International publication number: WO 2012/158130

(56) References cited:
- WO-A1-01/56628
- WO-A1-2004/067028
- US-A1- 2006 008 497

## Description

**Pertinent Art.** The herein invention is related to human and veterinary medicine and may be used in transplantation of cells, tissues and organs, as well as in implantation of various substances and devices, both of organic and inorganic nature, into recipient body.

**Prior Art.** The substances are known that are used for provision of implant biocompatibility with recipient body through chemico - therapeutic interference with recipient's immune system, substantially on a non - specific basis. Rapamycin, cyclosporin, antagonists of lymphocytes, and other representatives of cyclosporin series, may be referred to as examples of such substances [1].

The disadvantage of application of the substances hereinbefore is that immunosuppressive therapy aimed at destruction of recipient's own immunity can cause development of cancerous diseases. Reduction of immune system activity promotes increase of the number of cases of infections disease and acceleration of atherosclerosis development. Some of the patients die from the said complications rather than from transplant failure. In particular, the patients with transplanted heart die from the ischemic heart disease.

The substance is known that is used for provision of implant compatibility with recipient body which contains preliminary treated patient's serum, sodium adenosine triphosphate, potassium dihydrophosphate, as well as heat shock proteins. [2].

The disadvantage of application of the substance hereinbefore is that after thermal treatment of the initial serum during 20 - 45 minutes at 40 - 60°C and its further dilution, immunoglobulins of the serum may still remain active and identify the specific determinants on the surface of material. In such a case, the rate of their absorption on material surface will be higher than that of the serum albumin. Due to the said specific absorption of immunoglobulins on the surface of material, the mechanisms of body protection against exogenous material will be activated.

Thermal treatment of serum at 40 - 60°C during 20 - 45 minutes can result in polymerization of albumin molecules, which may have a substantial effect on the quality of implant treatment.

The substance most similar to the subject matter of the invention, which is used equivalently to the subject matter of the invention, contains serum albumin, sodium or potassium dihydrophosphate and sodium chloride solution in the following proportions, in per cent by mass: serum albumin - 0,1 - 6,0; sodium dihydrophosphate - 0,10 - 0,20; sodium chloride - 0,7 - 1,0; potassium dihydrophosphate - 0,10 - 0,20; and the remaining per cent is for water [3]. The said substance was chosen as an invention prototype.

The causes precluding attaining of the expected performance result from the known substance application are as follows: its composition includes certain levels of active high molecular (over 100 000 daltons) serum proteins. Among such proteins are class G immunoglobulins. They are responsible for identification of harmful substances in the body. The recipient's serum can contain immunoglobulins G specific to material surface, which may trigger the protective chain of implant rejection at contact with material surface. Besides, albumin heating during 20 - 45 minutes at 40 - 60°C can cause aggregation of its molecules. Albumin in such a state does not form a homogeneous film of inversibly absorbed protein on the surface of material and fails to perform its protective functions in full scope.

The substance that was chosen as an invention prototype is balanced only to the extent of composition of electrolytes. If it is used correctly for implant treatment, it is desirable to sustain its relevant osmolarity in order to prevent formation of aggregates and to avert edema or cell lysis in a case of its application in cell therapy.

It was shown experimentally in a large number of animals that the body reaction to the implant is still observed in 10 - 12% of cases. Though, this reaction is considerably weaker than in cases of untreated implant. Immunohistochemical analysis of tissue samples taken from the place of introduction of an implant treated by the substance chosen as an inventory prototype has demonstrated the presence of lymphocytes in tissues. It evidences that initial serum incubation in the said range of time and temperature results in polymerisation of the albumin molecules, whereupon they lose their ability to formation of a thin protective film on the implant surface Also, it evidences that neither incubation temperature conditions, no dilution of the initial serum, exclude ability of immunoglobulins class G to absorption on the implant surface, thus triggering reaction of foreign body identification by the recipient. For ensuring the fair effect of the adapted substance, it is desirable to extract albumin from recipient's serum *ex tempore* before treatment of the implant. However, all the existing techniques of serum albumin fractioning considerably complicate obtaining of the proposed substance and increase the probability of substance contamination with pathogenic microflora at the stage of its preparation.

The task underlying the herein invention is provision of abatement of the implant rejection reaction through due selection of qualitative and quantitative composition of the substance and facilitation of regeneration of the cells damaged during surgical intervention.

**Description of Invention.** The assigned task is solved owing to proper composition of the substance enhancing biocompatibility of implants with recipient body, which contains recipient serum albumin and, also, additionally contains amino acids, phosphorus and ions of magnesium, sodium, potassium and chlorine, with the following proportion of components: serum albumin - 0,1 - 9,9 g; alpha - ketoglutaric acid - 0,01 - 0,0176 g; histidine - 1 - 1,793 g; histidine hydrochloride monohydrate - 0,2 - 2,377 g; potassium hydroxide, potassium chloride - 0,05 - 0,0671 g; calcium chloride dehydrate - 0,0001 - 0,00022 g; magnesium chloride hexahydrate - 0,08 - 0,08132 g; mannitol - 0,5 - 5,547 g; sodium chloride - 0,8 - 8,087 g; tryptophan - 0,04 - 0,0408 g; water - in amount required for bringing the volume to 1 litre, with osmomolarity 280 - 310 mOsm/l. Method of preparation of the substance for enhancement of implant biocompatibility with recipient body, which includes mixing of the components preceded by thermal treatment of the recipient's serum albumin during 1 - 2 minutes at 94 - 95°C.

According to the method of preparation of the claimed substance, the serum should be subjected to thermal treatment and components should be mixed with further centrifugation of the mixture during 2 - 15 minutes at 1000 - 2500 rpm. Here, the supernatant fluid is used for preparation of the substance for implant surface treatment.

The claimed invention allows modification of the implant surface by the substance influencing in such a way the donor transplant/implant of either organic, or inorganic, nature that thereafter the latter is perceived as native by the relevant components of immune system.

The proposed substance increases biocompatibility of implants and facilitates their grafting in recipient body. Said substance modifies the exterior and interior surface of the implant and protects donor's cells, retards the process of implant rejection by diminishing the possibility of accumulation of the free radicals that can result in mutagenic changes in cells, and sustains pH required by physiological processes. The substance provides termination of recruiting by the implant surface the lymphocytes and macrophages that actively participate in the processes of inflammation and fibrous tissue formation around the implant.

The claimed substance can be applied in cell therapy, in transplantation of cells and tissues, in the processes of plasma sorption, lymph sorption and hemosorption, as well as in implantation of prostheses, cava filters, stents, microcircuits and chips, etc.; it allows prevention of forming contracture capsule around the implant.

Said substance can be prepared by addition of preliminary sterilized 0,9% aqueous sodium chloride solution of components at a room temperature to recipient blood serum. The components used for substance preparation are as follows: 0,176 g of commercially available preparation custodiol, which contains alpha - ketoglutaric acid; 27,93 g of histidine; 3,77 g of hydrochloride monohydrate; 0,671 g of potassium hydroxide, potassium chloride; 0,0022 g of calcium chloride dihydrate; 0,8132 g of magnesium chloride hexahydrate; 5,47 g of mannitol; 0,87 g of sodium chloride; 0,408 g of tryptophan; and water in amount required for bringing the volume to 1 liter, with osmomolarity 310 mOsm/l.

The preparation of implants for their contact with recipient body tissues provides their treatment for 5 - 10 minutes by the claimed substance at 20 - 25°C with the purpose of abating reaction of recipient tissues on exogenic material.

**Examples of Invention Embodiment.** Said invention may be demonstrated by examples of its embodiment (without method of preparation).

### Example 1.

The experiment studied the effect of the proposed substance on implant compatibility with recipient. The effect was estimated from measurements of the area of fibrous tissue around the implant in conventional units at a standardised slice. Decrease or absence of the fibrous tissue around the implant evidenced abatement of irritation, as well as higher implant propinquity with recipient body.

Male rabbits of Gray Giant breed, 0,5 - 2,0 years of age, weight 3,0 - 3,5 kg, were used in experiments. The Z - shaped stents made from the stainless steel (grade 316 L) wire of 0,18 mm in diameter and treated by the claimed substance were implanted to 70 animals. The implants were treated in accordance with the method claimed herein ***ex tempore*** before their introduction into abdominal aorta of a rabbit. The surgery was done under intravenous anaesthesia by Hexenal. Reaction of rabbit aorta wall on implantation of a stent treated by the claimed substance was studied (12 animals). The control group contained rabbits with implants introduced without preliminary treatment by any substance (12 animals) and with preliminary treatment by a substance taken as a prototype (12 animals). The rabbits that were not introduced implants (10 animals) were used as a kind of a norm. The animals were kept under normal conditions during 8 weeks after the surgery. After the end of experiment, the animals were put down by introduction of a lethal dose of hexenal. The histochemical analyses were performed in order to study the histological preparations of vessels. The preparations were stained by haematoxylin and eosin and using Van Gieson's technique. Morphometry measurements were carried out automatically using software, by Olympus microscope, at X 20 magnifications, with X 10 ocular. The neointimal thickness was estimated in conventional units at a standardised slice. The obtained results were analysed using methods of non - parametric statistics.

The average numerical data of measurements in 4 groups of animals with estimate reliability p < 0,001 are presented in Table 1.

**Table 1**

| Groups | Conventional Units |
|---|---|
| Initial aorta (n = 70). Norma | 138 ± 32 |
| Stainless steel stent (n = 10). Without treatment | 462 ± 82 |
| Stents treated in accordance with the prototype (n = 12). | 172 ± 35 |
| Stents treated by albumin obtained from rabbit blood serum by precipitation (n = 12). | 156 ± 28 |
| Stents treated by the claimed substance. Application of albumin with diluted rabbit blood serum to the stent after 2 minute thermal treatment at 95°C (n = 12). | 149 ± 46 |
| Stents treated in accordance with the claimed method. | 149 ± 46 |
| Stents treated by the claimed substance. Application of albumin with diluted rabbit blood serum to the stent after 1 minute thermal treatment at 95°C (n = 12). | 163 ± 32 |
| Stents treated by the claimed substance. Application of albumin with diluted rabbit blood serum to the stent after 1 minute thermal treatment at 94°C (n = 12). | 168 ± 34 |

The results of experiments show that the claimed substance, if it is used for preliminary treatment of implants, provides abatement of reaction of the implant surrounding tissues owing to implant surface modification and decreases the thickness of neointimal proliferation, which evidences the absence of inflammation processes and fibrous tissue formation around the implant.

### Example 2.

Animals (mice of 357BL strain) with simulated diabetes mellitus, induced by daily introduction of streptozotocin solution (40 mg/kg) five times a day, were injected suspension of insulin - producing cells in amount 15 10³/ml per mouse. The animals were divided into 7 groups containing 8 animal units each. First control group included animals that were not injected suspension of insulin - producing cells. The animals of the second group were injected suspension of insulin - producing cells without any treatment. The animals of the third group were injected suspension of insulin - producing cells preliminary treated in accordance with invention prototype. The animals of the fourth group were injected suspension of insulin - producing cells treated by the claimed substance. The animals of the fifth group were injected suspension of insulin - producing cells treated by mouse albumin obtained from mouse blood serum by chromatography on cellulose. The animals of the sixth group were injected suspension of insulin - producing cells treated by the claimed substance and prepared in accordance with the claimed method by thermal treatment at 95°C during 2 minutes. The animals of the seventh group were injected suspension of insulin - producing cells treated by the claimed substance and prepared in accordance with the claimed method by thermal treatment at 94°C during 1 minute. The glucose content in the blood of animals was measured through blood sampling from ophthalmic vein of an animal with its further analysis by automatic biochemical analyser Bio System (Spain). The results are presented in Table 2.

**Table 2**

| Groups | Glucose concentration (in mg/dl) |
|---|---|
| 1^{st} group, control; | 10,5 |
| 2^{nd} group without treatment; | 21,2 |
| 3 ^{rd} group; cells treated in accordance with invention prototype; | 12,7 |
| 4^{th} group; cells treated by the claimed substance; | 5,6 |
| 5^{th} group; cells treated by albumin obtained by partition chromatography; | 3,8 |
| 6^{th} group; cells treated by the claimed substance prepared in accordance with the claimed method (95°C during 2 minutes); | 3,5 |
| 7^{th} group cells treated by the claimed substance prepared in accordance with the claimed method at 94°C during I minute. | 5,6 |

The results obtained in the process of experiment evidence that preliminary treatment by the claimed substance and claimed method of its preparation preserve integrity and functionality of cells introduced into the animal body in the said manner. Preservation of cells, treated by the claimed substance after their introduction into the animal body, evidences suppression of the reaction of their rejection.

### Example 3.

This experiment studies data dealing animal body reaction on implantation of artificial materials. Mice of 357BL strain were intraperitoneally implanted under general anaesthesia by ketamine: carbon fabric Dnepr, cellophane, and medical grade silicone. The animals were divided into groups. The control group included animals that were implanted materials without their preliminary treatment (5 animal units per each material). The animals of the second group were implanted materials treated in accordance with invention prototype (5 animal units per each material). The animals of the third group were implanted materials treated by the claimed substance (5 animal units per each material). The body reaction was estimated by increase of glucose level in animal blood. The blood levels were measured automatically by biochemical analyser Bio System (Spain). The results are presented in Table 3.

**Table 3**

| Group | Implanted materials | Glucose level, in mg/dl |
|---|---|---|
| 1^{st} | Carbon fabric | 7,2 |
| | Cellophane | Animals died on the 7^{th} day |
| | Silicone | Animals died on the 7^{th} day |
| 2^{nd} | Carbon fabric | 3,8 |
| | Cellophane | 4,2 |
| | Silicone | 4,8 |
| 3^{rd} | Carbon fabric | 3,8 |
| | Cellophane | 4,0 |
| | Silicone | 4,2 |

Thus, the substance claimed demonstrates high efficiency in abatement of recipient tissue reaction on exogenic material and can be successfully applied in human and veterinary medicine in preparation of implants to contact with recipient body tissues.

### Example 4.

This experiment studied interaction of a probe made from silicon carbide with different surfaces. The following surfaces were prepared for experiment: gold, mica, glass, and silicon. The surfaces were studied by atomic - force microscope (AFM) Dimension 3000 Nano Scope IIIa (USA), using DNP - 20 probe with a nominal hardness of cantilever beam 0,06 N/g. The force of probe interaction with a surface was recorded in the range of nN (Newtons X 10⁻⁹). Then surfaces were treated by the claimed substance. The modified surfaces were studied again using AFM The results are presented in Table 4.

**Table 4**

| | Force of probe interaction with a substrate, in nN | | | |
|---|---|---|---|---|
| | Gold | Glass | Mica | Silicon |
| Pure substrate | 113 | 85 | No contacts with a surface | 117 |
| Substrate treated by the proposed substance (treatment at 95°C during 2 minutes) | 16 | 19 | 16 | 21 |
| Substrate treated by the substance in accordance with the proposed method (treatment at 94°C during 1 minute) | 52 | 34 | 16 | 38 |

**Industrial applicability.** The substance for enhancing implant biocompatibility with recipient body and method of its preparation were applied in mammary implant introduction to a patient with indications for replantation in relation to implant rejection The mammary implant was treated by the claimed substance with application of the claimed method of its preparation under sterile conditions, and then it was implanted to the patient. In two months after the surgery no reaction of aseptic inflammation or rejection was observed. Thus, the claimed substance demonstrates high efficiency in abatement of patient body reaction to the implant surface. The obtained results confirm that said treatment of an implant by the claimed substance abates reaction of tissues on exogenous materials and can be successfully applied in human and veterinary medicine for preparation of implants to contact with recipient body tissues.

### INFORMATION SORCES.

1. Patent of the Russian Federation No. 2214247, Bull. No.29, 2003;
2. Patent of Ukraine No. 20638, Bull. No.2, 2007;
3. Patent of Ukraine No. 56960, Bull. No.7, 2005.

## Claims

1. Substance for enhancement of implant biocompatibility with recipient body, which contains serum albumin, sodium chloride, sodium dihydrophosphate, potassium dihydrophosphate and water, **characterized in that** it contains recipient serum albumin and, also, additionally contains amino acids, phosphorus and ions of magnesium, sodium potassium and chlorine, with the following proportion of components:
| | |
|---|---|
| serum albumin | 0,1 - 9,9 g; |
| alpha - ketoglutaric acid | 0,01 - 0,0176 g; |
| histidine | 1,793 g; |
| histidine hydrochloride monohydrate | 0,2 - 2,377 g; |
| potassium hydroxide, potassium chloride | 0,05 - 0,0671 g; |
| calcium chloride dihydrate | 0,0001 - 0,00022 g; |
| magnesium chloride hexahydrate | 0,08 - 0,08132 g; |
| mannitol | 0,5 - 5,547 g; |
| sodium chloride | 0,8 - 8,087 g; |
| tryptophan | 0,04 - 0,0408 g; |
water in amount required for bringing the volume to 1 liter, with osmomolarity 280 - 310 mOsm/l.

2. Method of preparation of the substance according to claim 1, which includes mixing of the components, and the recipient serum albumin is subjected to thermal treatment during 1 - 2 minutes at 94 - 95°C prior to mixing.

## Patentansprüche

1. Substanz zur Verbesserung der biologischen Implantatkompatibilität mit dem Empfängerkörper, welche Serumalbumin, Natriumchlorid, Natriumdihydrogenphosphat, Kaliumdihydrogenphosphat und Wasser umfasst, **dadurch gekennzeichnet, dass** es Empfängerserumalbumin enthält und außerdem zusätzlich Aminosäuren, Phosphor und Magnesium-, Natrium-, Kalium- und Chlorionen enthält, mit den folgenden Anteilen an Komponenten:
| | |
|---|---|
| Serumalbumin | 0,1 - 9,9 g; |
| alpha-Ketoglutarsäure | 0,01 - 0,0176 g; |
| Histidin | 1,793 g; |
| Histidinhydrochlorid-monohydrat | 0,2 - 2,377 g; |
| Kaliumhydroxid, Kaliumchlorid | 0,05 - 0,0671 g; |
| Calciumchlorid-dihydrat | 0,0001 - 0,00022 g; |
| Magnesiumchlorid-hexahydrat | 0,08 - 0,08132 g; |
| Mannit | 0,5 - 5,547 g; |
| Natriumchlorid | 0,8 - 8,087 g; |
| Tryptophan | 0,04 - 0,0408 g; |
Wasser in einer Menge, die erforderlich ist, um das Volumen auf 1 Liter zu bringen, mit einer Osmolarität von 280 - 310 mOsm/l.

2. Verfahren zur Herstellung der Substanz nach Anspruch 1, welches das Mischen der Komponenten und die 1- bis 2-minütige thermale Behandlung des Empfängerserumalbumins bei 94 - 95°C vor dem Mischen umfasst.

## Revendications

1. Substance pour l'amélioration de la biocompatibilité d'un implant avec le corps d'un receveur, qui contient de la sérum-albumine, du chlorure de sodium, du dihydrogénophosphate de sodium, du dihydrogénophosphate de potassium et de l'eau, **caractérisée en ce qu'**elle contient de la sérumalbumine de receveur et, en outre, contient également des acides aminés, du phosphore et des ions de magnésium, de sodium, de potassium et de chlore, avec les proportions de composants suivantes :
| | |
|---|---|
| sérum-albumine | 0,1-9,9g; |
| acide alpha-cétoglutarique | 0,01-0,0176 g ; |
| histidine | 1,793 g ; |
| chlorhydrate d'histidine monohydraté | 0,2 - 2,377 g ; |
| hydroxyde de potassium, chlorure de potassium | 0,05 -0,0671 g ; |
| chlorure de calcium dihydraté | 0,0001 - 0,00022 g ; |
| chlorure de magnésium hexahydraté | 0,08 -0,08132 g ; |
| mannitol | 0,5 - 5,547 g ; |
| chlorure de sodium | 0,8 - 8,087 g ; |
| tryptophane | 0,04 - 0,0408 g ; |
eau en quantité requise pour amener le volume à 1 litre, avec une osmolarité de 280 à 310 mOsm/l.

2. Procédé de préparation de la substance selon la revendication 1, qui comprend le mélange des composants, et la sérum-albumine de receveur est soumise à un traitement thermique pendant 1 à 2 minutes à 94 à 95 °C avant mélange.
